# EUROPEAN PATENT APPLICATION

(11) **EP 1 050 586 A1**
(43) Date of publication of application: **08.11.2000**
(21) Application number: 99901113.3
(22) Date of filing: 20.01.1999
(51) Int. Cl.: C12N 15/86, A01K 67/027, C12N 5/10

(54) **METHOD FOR TRANSFERRING GENE INTO GERM CELL**

(30) Priority: 28.01.1998 JP 1562998
(71) Applicant: Takara Shuzo Co, Ltd., Kyoto-shi, Kyoto 612-8061 (JP)
(72) Inventor: UENO, Mitsuhiro, Kusatsu-shi, Shiga 525-0025 (JP); KONISHI, Haruko, Kyoto-shi, Kyoto 601-8412 (JP); MORISHITA, Mio, Otsu-shi, Shiga 520-0043 (JP); YUKI, Atsushi, Saitama 330-0023 (JP); ASADA, Kiyozo, Shiga 520-3333 (JP); KATO, Ikunoshin, Kyoto 611-0028 (JP)
(74) Representative: Grund, Martin, Dr.
(86) International application number: JP9900177
(87) International publication number: WO9938991

(57) **Abstract**

Germ cells having a desired foreign gene integrated thereinto; a convenient method for acquiring a vertebrate carrying these cells; and a method for constructing a novel vertebrate line by using these cells and the vertebrate. Namely, a method for transferring a foreign gene into the testes of a vertebrate which involves the step of decreasing a testicular cell number and the step of inoculating a recombinant virus carrying the foreign gene into the testes; the vertebrate having the foreign gene transferred thereinto by the above method; testicular cells obtained from the vertebrate and having the foreign gene transferred thereinto; a vertebrate obtained by mating the above vertebrate and carrying the transferred foreign gene; and a vertebrate obtained by artificial fertilization with the use of the testicular cells and carrying the transferred foreign gene; and a vertebrate obtained by artificial fertilization with the use of the testicular cells and carrying the transferred foreign gene.

## Description

### Technical Field

The present invention relates to a method for transferring a gene into a germ cell. More specifically, the present invention relates to a method for transferring a gene into a vertebrate germ cell and a method for producing a transgenic animal using a germ cell with a transferred gene obtained by said method. The method of the present invention is useful in the field of medicine, pharmacology, stockbreeding and the like.

### Background Art

The recent development in techniques for gene technology allowed the production of a transgenic animal, which is an animal in which a gene is modified, for example, by transferring a foreign gene or by deleting a certain gene. The techniques for producing a transgenic animal are used as experimental techniques for elucidating a function of a gene. The techniques are also used as means for obtaining a model animal for a disease state to be used in assessing efficacy of a pharmaceutical as well as a livestock having an excellent phenotype.

The most common method for producing a transgenic animal comprises injecting a DNA directly into a fertilized ovum by using a micro-manipulator or the like, transplanting the fertilized ovum into a surrogate mother, and then obtaining a grown individual [Gordon, J.H. et al., Proc. Natl. Acad. Sci. USA, 77:7380-7384 (1980)]. This method requires extremely precise operations including handling a fertilized ovum ex vivo and injecting a DNA into the fertilized ovum. Thus, successful results are expected only when an expert researcher conducts the method. In addition, since the injected DNA is not actively integrated into a chromosome, efficiency of obtaining an individual carrying the foreign gene is low.

Thus, it is required to treat a number of fertilized ova in order to accomplish the purpose certainly. Such a requirement brings problems such as a burden in operations and a cost for purchasing animals for collecting fertilized ova. Under these circumstances, various methods as described below have been designed and tested.

An attempt to transfer a foreign gene into an early embryo by using a retrovirus vector in order to effectively integrate the foreign gene into a chromosome has been reported [Bowen, R.A. et al., Molec. Reprod. Dev., 40:386-390 (1995)].

Also, an attempt to transfer a foreign gene into a fetus by intravenously injecting a mixture of a basic polyamine and a plasmid into a pregnant female mouse has been reported [Terada et al., Nature Genetics, 9:243-248 (1995)].

On the other hand, a method for producing a transgenic animal has been attempted. This method is based on a principle in which a genetically modified sperm is first produced, which is then used to produce a fertilized ovum. For example, a method in which testicular cells are destroyed by physical or chemical treatment, and then genetically modified spermatogonia are transplanted into a seminiferous tubule has reported [Brinster, R.L. et al., Proc. Natl. Acad. Sci. USA, 91:11303-11307 (1994); Nature Med., 2:693-696 (1996)].

Furthermore, the following attempt has been made. A circular or linear plasmid DNA containing a foreign gene is co-precipitated with calcium phosphate. The precipitate is then administered into a testis by using a liposome method to transfer the foreign gene into a testicular stem cell or a sperm [Tada, et al., Animal Biotechnol., 5:19-31 (1994); Takahashi et al., Nippon Chikusan Gakkai (Japanese Society of Animal Science) Abstract, 2x-8 (1996)].

The methods as described by Brinster et al. and Bowen et al. principally aim at obtaining a genetically modified individual with high efficiency. However, these techniques require high level of experimental techniques including maintenance of early stage embryos, gene transfer with viruses, and transplantation of cells into a testicular seminiferous tubule. The techniques also require expensive equipment for enabling the techniques. Therefore, it is impossible to conduct them with usual experimental equipment.

The methods as described by Tada et al., Takahashi et al. and Terada et al. are convenient because the methods do not require equipment as described above. However, the administered foreign gene is not actively integrated into the chromosome of the target germ cell in these methods. This results in little possibility of transmission of the gene to offspring although the existence of the gene may be observed transiently. In other words, these methods are not suitable for obtaining a germ cell into which a biologically functional gene of interest is integrated, or a transgenic animal originated from the cell.

### Objects of Invention

One object of the present invention is to provide a convenient method for obtaining a germ cell into which a foreign gene of interest is integrated and a vertebrate carrying said cell.

Another object of the present invention is to provide a method for producing a genetically novel vertebrate line utilizing said germ cell or said vertebrate.

### Summary of Invention

The present inventors have found that, in a gene transfer into testicular cells which comprises inoculating a recombinant virus into a testis of a vertebrate, the testicular cell with a transferred gene can be obtained readily and efficiently by combining inoculation of the virus and treatment for decreasing a testicular cell number. Thus, the present invention has been completed.

Thus, the first invention of the present invention relates to a method for transferring a gene into a vertebrate testicular cell. The method is characterized in that it comprises decreasing a testicular cell number and inoculating a recombinant virus carrying a foreign gene into a testis. A chemical procedure, a physical procedure and a biological procedure and the like can be used for decreasing the testicular cell number. Particularly, a chemical procedure in which an alkylating agent is used is preferable. Additionally, the recombinant virus is preferably inoculated into the testis during a restoring period for the testicular cells.

A recombinant virus capable of integrating into a chromosome can be used as the recombinant virus in the present invention. Preferably, a recombinant retrovirus can be used.

The second invention of the present invention relates to a vertebrate in which a foreign gene is transferred into its testicular cell. The vertebrate is characterized in that the foreign gene is transferred by the method of the first invention.

The third invention of the present invention relates to a testicular cell with a transferred foreign gene. The cell is characterized in that it is obtained from the vertebrate of the second invention.

Furthermore, the fourth invention of the present invention relates to a vertebrate with a transferred foreign gene. The vertebrate is characterized in that it is obtained by mating the vertebrate of the second invention or artificial fertilization using the testicular cell of the third invention.

### Detailed Description of the Invention

A recombinant virus is used as a vector in the method for transferring a gene into a testicular cell of the present invention. A virus vector capable of integrating a gene of interest into a chromosome of a target cell can be preferably used in the present invention. For example, the virus vectors which can be used include, but are not limited to, a retrovirus vector, a human immunodeficiency virus (HIV) vector, an adeno-associated virus vector. Furthermore, the virus vector of the present invention is preferably replication-defective such that the vector does not repeat unlimited infection.

Known replication-defective retrovirus vectors can be used in the method for transferring a gene of the present invention. For example, the vectors which can be used include, but are not limited to, a retrovirus vector such as MFG (ATCC No. 68754), α-SGC (ATCC No. 68755), pLRNL [Virology, 171:331 (1989)] and pBabe [Nucleic Acids Research, 18:3587-3596 (1990)], or a modified vector derived therefrom. In addition, the vector can be prepared as a virus supernatant containing virus particles into which the vectors are packaged by using a known packaging cell line such as PG13 (ATCC CRL-10686), PG13/LNc8 (ATCC CRL-10685), PM317 (ATCC CRL-9078), GP+E-86 (ATCC CRL9642) and GP+envAm-12 (ATCC CRL9641).

There is no limitation regarding the foreign gene to be transferred into a germ cell by the method of the present invention. Any gene of which the transfer is desired may be used. For example, a gene encoding an enzyme or another protein, a gene encoding a functional nucleic acid molecule such as an anti-sense nucleic acid, a ribozyme or a decoy can be transferred. There is also no limitation regarding the origin of the gene. The gene may be a gene derived from an organism of the same species as that of the germ cell into which the gene is to be transferred, a gene derived from a heterologous organism, a chemically synthesized gene, or a combination thereof. Furthermore, a regulatory element such as a promoter, a terminator and an enhancer suitable for regulating the gene expression may be added to the gene. In the present invention, the gene is used being incorporated into the above-mentioned virus vector.

The method for transferring a gene of the present invention comprises inoculating a recombinant virus containing a foreign gene to be transferred into a testis. The inoculation of the virus is conducted, for example, by injection into the testis using a syringe under anesthesia rather than a surgery such as a ventrotomy.

It is known that the ratio of the number of target cells to the number of viral particles (or titer of the virus) is a factor that greatly influences the infection efficiency in the infection of cells with a virus. For example, the present inventors conducted a natural mating for five weeks. In the natural mating, ten male mice were injected with 50 µl of a retrovirus (1 x 10⁴ cfu/ml) having an incorporated foreign gene at each of the testes. They were then housed together with eight weeks old normal female mice in one-to-one correspondence from Monday to Friday every week. Transgene detection by PCR analysis conducted on a total of 534 offspring from the fifty female mice revealed no offspring carrying the foreign gene used.

In order to solve such a problem, it is desirable to inoculate a recombinant virus with a high titer. The titer of the recombinant virus depends on the producer cell used for producing the virus. It is not practically easy to construct a producer cell that produces high-titer retrovirus vector. In the method for transferring a gene of the present invention, incorporation of a step of decreasing a testicular cell number has solved the problem.

As used herein, the testicular cell refers to a germ cell that exists in a testis. A gene is transferred into a testicular cell (including, for example, a spermatogonium, a spermatocyte, a spermatid and a sperm) by the method of the present invention.

As a procedure for decreasing a testicular cell number, a chemical procedure, a physical procedure or a biological procedure can be used alone or in combination. The chemical procedure refers to a procedure that decreases the testicular cell number by administering a chemical agent. For example, a DNA synthesis inhibitor, an RNA synthesis inhibitor, a DNA polymerase inhibitor, a mitotic inhibitor, an alkylating agent, a contraceptive agent or the like can be used. An X-ray irradiation or a γ-ray irradiation, for example, can be used as a physical procedure. Furthermore, a protein involved in proliferation or replication of a cell, for example, can be utilized for a biological procedure. Any of the various procedures as described above can be used to decrease the testicular cell number. Among these, a chemical procedure which does not require a specialized equipment or operation and of which the activity is readily controlled is preferably used for decreasing the testicular cell number in the present invention.

Procedures for chemically decreasing a testicular cell number include, for example, a procedure in which an alkylating agent is used. The alkylating agents that can be used in the method of the present invention include, for example, mitomycin C, nitrogen mustard-N-oxide, TESPA, melphalan, carboquone, nitrosourea derivatives, cyclophosphamide, busulfan and the like.

The method for transferring a gene of the present invention comprises decreasing a testicular cell number to a degree that allows the cell number to restore to the level prior to the treatment. The treatment for decreasing the testicular cell number as described above deprives the testis of mature cells. Thereafter, remaining spermatogonia begin to divide and proliferate actively to restore the cell number to the level prior to the treatment after a certain period of time. These events can be confirmed by examining the testis, microscopically observing the tissue sections of the testis, analyzing the cell cycle using FACS (Fluorescence Activated Cell Sorting) and conducting in vivo mating experiments in time course on the animal individual subjected to the treatment for decreasing the testicular cell number. These experiments reveal the relationship between the treatment for decreasing the testicular cell number and the time-course of the cell number, from decrease to restoration, in detail.

Inoculation of a recombinant virus containing a foreign gene into a testis of which the cell number has been decreased as described above results in highly efficient infection of testicular cells with the virus. Furthermore, spermatogonia, spermatocytes, spermatids and sperms with the transferred foreign gene are accumulated in the testis as the testicular cell number restores. Preferably, the restoring period during which the remaining cells are actively proliferating is selected for the administration of the virus. It is known that a retrovirus, which is frequently used as a vector for transferring a gene into cells, preferentially infects diving cells. Therefore, the restoring period provides particularly preferable environment for the infection with the virus.

Inoculation of high-titer recombinant virus can further increase the efficiency of gene transfer. Such a high titer can be accomplished, for example, by preparing a virus supernatant using a selected packaging cell capable of producing high-titer virus. Alternatively, a virus concentrated by an appropriate method may be used. For example, viral particles of a VSV-G vector, a retrovirus vector of so-called pseudo-type, can be concentrated by centrifugation [Proc. Natl. Acad. Sci. USA, 90:8033-8037 (1994)].

The present invention provides a convenient method for transferring a foreign gene into a vertebrate germ cell, which does not involve a surgery or handling of a germ cell ex vivo. A male vertebrate individual carrying germ cells with a transferred foreign gene can be obtained by using the present invention. In addition, a fertilized ovum derived from a germ cell with a transferred gene, as well as a vertebrate individual developed from the fertilized ovum can be obtained by mating the resulting male vertebrate individual with a female individual. Techniques other than the mating can be used to obtain a fertilized ovum. For example, artificial fertilization in which an ovum is fertilized in vitro with testicular cells (e.g., sperms) collected from the male vertebrate individual as described above can be used to produce a fertilized ovum. The fertilized ovum produced by artificial fertilization can be developed into an animal individual by transplanting it to an appropriate surrogate mother.

The thus-obtained animal individuals contain one having a transferred foreign gene (i.e., a modified gene) on only one of the chromosomes in its cell (a hetero-individual). Such an individual can be selected by examining if its genome contains the foreign gene using a known method such as a PCR analysis or a hybridization method. A transgenic animal stably carrying a transferred gene and exhibiting a phenotype derived from the gene can be obtained by selecting a homo-individual, an individual having a modified gene on both of the chromosomes, from the offspring obtained by mating the hetero-individuals.

The present invention can be applied to any animal species except for a human. For example, it can be applied to rodents (e.g., mice, rats, etc.) which are frequently used for researches, as well as livestock and poultry (e.g., domestic cattle such as milk cows and beef cattle, horses, pigs, birds such as chickens, etc.). A transgenic animal which is useful as an experimental animal or which has an excellent phenotype as a domestic animal can be produced readily and at a low cost by using the method of the present invention.

A male vertebrate individual, in which a foreign gene of interest is transferred into its testicular cells by the method of the present invention, produces sperms having the gene integrated into their chromosomes. Therefore, it is expected that the method serves as, for example, means for producing a stud bull or a studhorse for producing valuable calves or foals. Offspring carrying the gene can be produced by mating the male animal.

### Brief Description of Drawings

Figure 1 shows analytical data for testicular cells from mice administered with busulfan using FACS. A, B and C in the figure represents analytical results for testicular cells obtained 1, 3, and 5 weeks after the busulfan administration, respectively.
Figure 2 shows reproducibility of male mice administered with busulfan. The pregnancy rate in the figure represents the ratio of pregnant female mice. A, B and C in the figure represent results for control mice, mice administered with 20 or 30 mg/kg of busulfan, respectively.

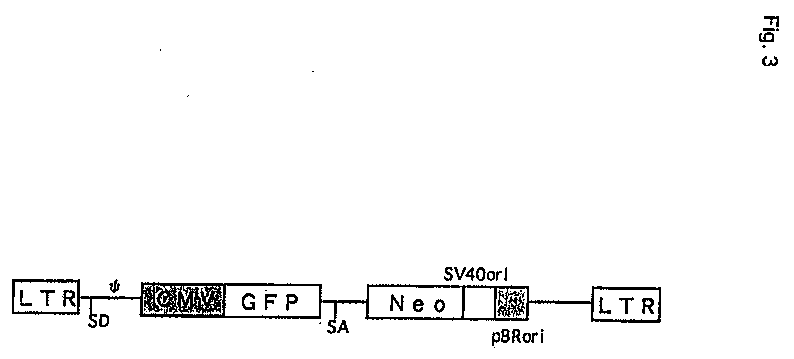

Figure 3 depicts a region of the vGL2 viral genes to be integrated into a chromosome of an infected cell contained in a plasmid pGL2. In the figure, LTR represents a LTR (Long Terminal Repeat) from Moloney murine leukemia virus, SD represents a splice donor site, ϕ represents a packaging signal, CMV represents an early enhancer-promoter derived from cytomegalovirus, GFP represents a green fluorescence protein gene, SA represents a splice acceptor site, Neo represents a neomycin phosphotransferase gene, SV40ori represents a replication origin from SV40, and pBR322ori represents a replication origin from pBR322, respectively.
Figure 4 shows results obtained by FACS analysis of GFP-expression in testicular cells from mice inoculated with the vGL2 virus.
Figure 5 shows results obtained by FACS analysis of GFP-expression in sperms collected from epididymes of mice inoculated with the vGL2 virus.

### Examples

The following Examples illustrate the present invention in more detail, but are not to be construed to limit the scope thereof.

### Example 1

Changes in murine testicular cells caused by intraperitoneal administration of busulfan

### (1) Effects on weight of testis

5, 10, 20 or 40 mg/kg of busulfan (1,4-butanediol dimethanesulfonate, Wako Pure Chemical Industries) was administered intraperitoneally to MCH (ICR) male mice of four weeks old (Clea Japan). The weight of testes removed from the mice 1, 2, 3, 4 and 5 weeks after the administration was measured and compared with the weight of testes from control mice administered with saline.

As a result, reduction in weight was observed for mice administered with 20 or 40 mg/kg of busulfan as shown in Table 1. In addition, a tendency to restore weight five weeks after administration was observed for mice administered with 20 mg/kg of busulfan.

**Table 1**

| Effects of busulfan administration on weight of testes of mice | | | | | |
|---|---|---|---|---|---|
| Amount of busulfan administered (mg/kg) | Time after drug administration (weeks) | | | | |
| | 1 | 2 | 3 | 4 | 5 |
| Control | 93.9 mg | 129.5 mg | 123.4 mg | 140.3 mg | 145.6 mg |
| 5 | 98.2 mg | 124.5 mg | 105.0 mg | 114.7 mg | 97.4 mg |
| 10 | 75.8 mg | 113.3 mg | 106.1 mg | 112.4 mg | 115.1 mg |
| 20 | 101.5 mg | 96.3 mg | 78.8 mg | 71.4 mg | 87.3 mg |
| 40 | 100.3 mg | 89.0 mg | 46.9 mg | 45.5 mg | 34.2 mg |
| Age of mice (weeks) | 5 | 6 | 7 | 8 | 9 |

### (2) Effects on body weight

10, 20, 30 or 40 mg/kg of busulfan was administered intraperitoneally to MCH (ICR) male mice of four weeks old. Change in body weight was measured until seven weeks after the administration and compared with the body weight of control mice administered with saline. Five mice per group were used for each of the administration groups and the control group.

As a result, the busulfan administration within the range of the amount tested did not affect the body weight of mice at all as shown in Table 2. In addition, no change in appearance was observed for the mice.

**Table 2**

| Effects of busulfan administration on body weight of mice | | | | | | |
|---|---|---|---|---|---|---|
| Age of mice (weeks) | Time after administration (weeks) | Amount of drug administered | | | | |
| | | Control | 10 mg/kg | 20 mg/kg | 30 mg/kg | 40 mg/kg |
| 4 | 0 | 24.6±0.3 | 25.6±0.7 | 24.0±0.6 | 23.9±0.6 | 23.9±0.6 |
| 5 | 1 | 31.1±1.4 | 31.2±1.2 | N.D. | N.D. | N.D. |
| 6 | 2 | 32.8±2.0 | 33.8±1.9 | N.D. | N.D. | N.D. |
| 7 | 3 | 35.4±2.1 | 35.6±1.6 | N.D. | N.D. | N.D. |
| 8 | 4 | 36.0±2.1 | 36.3±1.3 | 36.7±1.4 | 34.7±1.5 | 36.2±0.9 |
| 9 | 5 | 35.2±2.5 | 37.3±1.6 | 36.7±1.6 | 35.4±1.3 | 36.3±0.7 |
| 10 | 6 | 34.9±2.3 | 36.5±1.3 | 36.1±1.6 | 34.4±1.7 | 35.6±1.5 |
| 11 | 7 | 35.1±2.2 | 36.1±1.0 | 36.8±2.3 | 34.7±1.3 | 36.8±2.0 |
| Expressed as mean body weight of five mice per group ± standard deviation (in gram). N.D.: not determined. | | | | | | |

### (3) Results of microscopic observation on tissue sections of testes and epididymes

20, 30 or 40 mg/kg of busulfan was administered intraperitoneally to MCH (ICR) male mice of four weeks old. Tissue sections were prepared from testes and epididymes removed from the mice ten weeks after administration. These sections were subjected to HE stain (hematoxylin-eosin stain) and then observed microscopically. The state of the tissue and/or the cells was compared with that of control mice administered with saline. Five mice per group were used for each of administration groups and control group.

As a result, both of the testes and epididymes of mice in the group administered with 20 mg/kg or 30 mg/kg of busulfan recovered to the state almost identical with that of the control mice as shown in Table 3.

**Table 3**

| Results of microscopic observation on tissue sections of testes and epididymes from mice administered with busulfan | | | | | |
|---|---|---|---|---|---|
| Amount of drug administered | Microscopic observation | | | | Assessment (State of restoration) |
| | Testicular cells | | Epididymal sperms | | |
| | Seminiferous tubule | Spermatogonium | Epididymal head | Epididymal tail | |
| Control | Filled | Filled | Somewhat filled | Fulfilled | Normal |
| 20 mg/kg | Filled | Filled | Somewhat filled, partially with hollow tubes | Fulfilled | Restored |
| 30/mg/kg | Somewhat filled, with detached tubes | Lack -somewhat filled | Somewhat filled, partially with hollow tubes | Trace -fulfilled | Restored |
| 40 mg/kg | Somewhat filled, with detached tubes | Lack -somewhat filled | Somewhat filled, partially with hollow tubes | Cavitation - somewhat fulfilled | Not restored with partial restoration |
| Assessed based on results of microscopic observation on tissue sections of testes and epididymes from five mice per group. | | | | | |

### (4) FACS analysis of testicular cell population intraperitoneally administered with busulfan

20 or 30 mg/kg of busulfan or saline (for control group) was administered intraperitoneally to MCH (ICR) male mice of four weeks old (six mice per group). A pair of testes were removed from a mouse from each group 1, 2, 3, 4, 5 and 6 weeks after administration. Testicular cells were then treated according to the following procedure. First, after the tunicae of the testes were removed, collagenase I (Gibco) was added thereto to a final concentration of 1 mg/ml for treatment at 37°C for 15 minutes. The mixture was washed, and 1 ml of 0.25% trypsin/EDTA (Gibco) was then used to disperse cell masses. After 100 micron or larger cell masses were removed by filtration, cells were fixed with 1% paraformaldehyde followed by 70% ethanol. The cells were washed with phosphate buffered saline (PBS), suspended in PBS, treated with 2 mg/ml of RNase A (Sigma), and then subjected to a DNA staining with 0.05 mg/ml of propidium iodide (Sigma). Cell masses were removed using a 30-micron mesh immediately before the FACS analysis. The DNA content in the cell was analyzed using FACSVantage (Becton Dickinson) at an excitation wavelength of 488 nm and a emission wavelength of 564-606 nm.

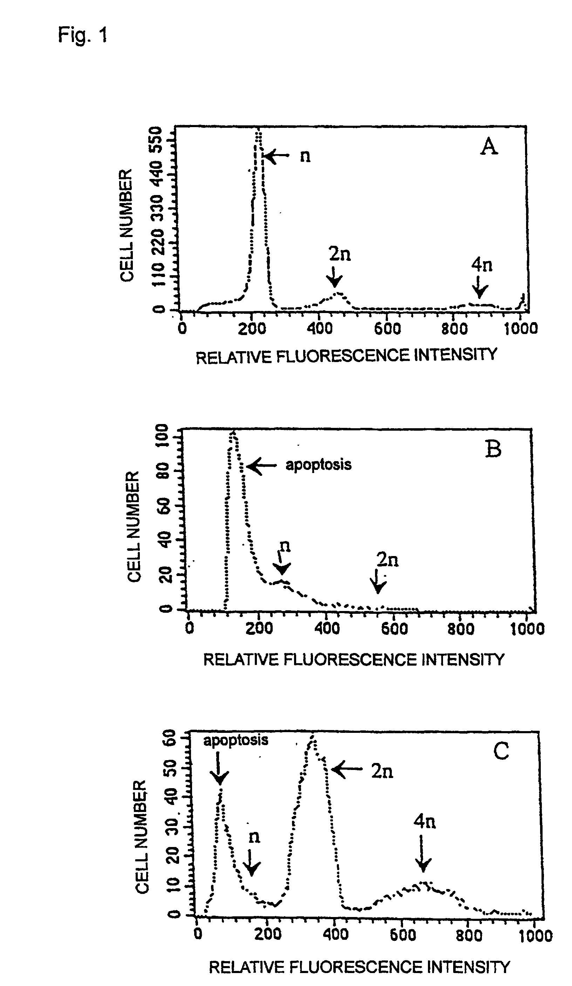

The FACS analysis can be used to determine the numbers of the following cells. Spermatogonia in G2 phase and M phase as well as primary spermatocytes which have a DNA content of 4n; spermatogonia in G1 phase and secondary spermatocytes which have a DNA content of 2n; spermatids and sperms which have a DNA content of n; and apoptotic cells which have a DNA content lesser than the above. The values determined from the analytical data are indicated in Table 4. FACS data for testicular cells obtained from mice 1, 3 and 5 weeks after the administration of 20 mg/kg of busulfan are shown in Figure 1 as a representative.

Figure 1 shows that the apoptosis in most of the testicular cells is observed at three weeks after administration, whereas the ratio of the cells with 2n is increased five weeks after administration. In addition, the results shown in Table 4 reveal the following. The least total cell number in the testis was observed about four weeks after the busulfan administration. The regeneration of the cells begins thereafter. The number of the cells with 2n is first increased during the course of regeneration of the cells.

**Table 4**

| Changes in testicular cell type in mice after intraperitoneal busulfan administration | | | | |
|---|---|---|---|---|
| Amount of busulfan administered | Time after busulfan administration (weeks) | Cell number (x 10⁵/testis) | | |
| | | n | 2n | 4n |
| Control | 0 | 53.4 | 12.4 | 7.8 |
| | 1 | 103.6 | 17.7 | 16.3 |
| | 2 | 97.8 | 10.7 | 9.4 |
| | 3 | 87.6 | 8.9 | 4.3 |
| | 4 | 132.6 | 20.8 | 13.3 |
| | 5 | 49.8 | 206.1 | 21.4 |
| | 6 | 93.5 | 376.0 | 51.2 |
| 20 mg/kg | 0 | 53.4 | 12.4 | 7.8 |
| | 1 | 72.2 | 11.7 | 4.7 |
| | 2 | 110.0 | 12.7 | 3.6 |
| | 3 | 64.0 | 13.2 | 1.6 |
| | 4 | 36.6 | 14.8 | 3.8 |
| | 5 | 4.3 | 47.4 | 9.3 |
| | 6 | 4.7 | 153.6 | 17.0 |
| 30 mg/kg | 0 | 53.4 | 12.4 | 7.8 |
| | 1 | 75.4 | 10.1 | 4.7 |
| | 2 | 67.6 | 7.8 | 0.7 |
| | 3 | 22.8 | 7.5 | 0.7 |
| | 4 | 4.7 | 7.4 | 0.6 |
| | 5 | 2.5 | 30.2 | 5.7 |
| | 6 | 6.1 | 35.7 | 14.4 |
| Results are expressed as mean values of respective cell numbers for two mice. | | | | |

| | | | | |
|---|---|---|---|---|
| n: spermatids and/or sperms. | | | | |
| 2n: spermatogonia in G1 phase and/or secondary spermatocytes. | | | | |
| 4n: spermatogonia in G2 and M phases and/or primary spermatocytes. | | | | |

### (5) Decrease in reproducibility in male mice caused by intraperitoneal busulfan administration and determination of restoring period

20 or 30 mg/kg of busulfan was administered intraperitoneally to MCH (ICR) male mice of four weeks old (six mice per group). The mice were subjected to natural mating. The natural mating was conducted for five days a week (from Monday to Friday) from one to ten weeks after administration. They were mated with female mice, which were replaced every week. The pregnancy ratio of the female mice was determined and the reproducibility of the male mice administered with busulfan was compared with that of the control group administered with saline.

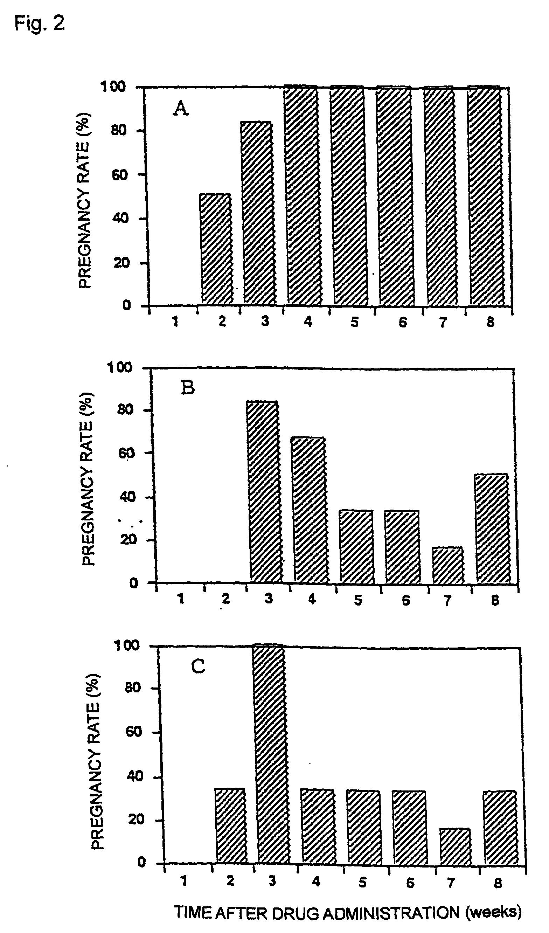

As a result, decrease in reproducibility (4-7 weeks after the busulfan administration) and restoration thereof (8 weeks after the busulfan administration) were observed for the groups intraperitoneally administered with 20 mg/kg or 30 mg/kg of busulfan as shown in Figure 2. It is known that it requires about 3-6 weeks for a spermatogonium to become a spermatocyte, a spermatid, and finally a mature sperm in a mouse. The restoration of testicular cells after the busulfan administration is observed five weeks after the drug administration or later in the results of microscopic observation of tissue sections as described in Example 1-(3) and (4) as well as the analytical results of change in testicular cell population using FACS. The fact that the restoration of reproducibility in male mice was observed three weeks later is consistent well with the period required for the formation of a mature sperm from a spermatogonium.

### Example 2

### Transfer of a foreign gene into murine testicular cells and its expression

### (1) Construction of a recombinant retrovirus

A plasmid pGreen Lantern-1 (Life Technologies Oriental) contains a gene encoding green fluorescence protein (GFP). In the gene, codons are optimized for a human and the amino acid serine encoded at position 65 from the N-terminus is replaced by threonine (S65T). The plasmid was digested with SspI (Takara Shuzo) and applied to 1% agarose gel electrophoresis. A DNA fragment corresponding to approximately 1.9 kb was recovered. On the other hand, a plasmid pZIP-NeoSV(X)I [Cell, 37:1053-1062 (1984)) was digested with BamHI (Takara Shuzo). The termini were blunt-ended using a DNA Blunting Kit (Takara Shuzo), and then dephospholylated with alkaline phosphatase (Takara Shuzo). The resulting plasmid DNA was mixed and ligated with the above-mentioned DNA fragment of approximately 1.9 kb, and the mixture was then introduced into E. coli JM109 (Takara Shuzo). Plasmids harbored by the resulting transformants were examined to select one that contains only one molecule of the fragment of approximately 1.9 kb. The thus-obtained plasmid was designated as a plasmid pGL2. Viral particles derived from the plasmid can infect cells and integrate the GFP gene into the chromosome of the infected cells. The region of the retroviral genes to be integrated into the chromosome of the infected cells contained in the plasmid pGL2 is shown in Figure 3.

### (2) Preparation of vGL2 virus supernatant

A virus derived from the plasmid pGL2 is designated as a vGL2 virus. A supernatant containing the virus was prepared by using BOSC23 as packaging cell line [Proc. Natl. Acad. Sci. USA, 90:8392-8396 (1993)]. Briefly, 1 x 10⁷ BOSC23 cells were cultured overnight in 10 ml of Dulbecco's Modified Eagle's Medium (DMEM, Iwaki Glass) containing 10% fetal calf serum (FCS, Dainippon Pharmaceutical), 50 units/ml of penicillin and 50 µg/ml of streptomycin (both from Gibco) in a 10-cm diameter gelatin-coated cell culture dish (Iwaki Glass) (all of the DMEMs used in the procedures hereinbelow contain 50 units/ml of penicillin and 50 µg/ml of streptomycin). After the medium was exchanged for 10 ml of fresh medium, transfection was carried out by calcium phosphate coprecipitation method using 50 µg of plasmid pGL2. The medium was exchanged for 10 ml of fresh medium after eight hours and for 5 ml of fresh medium after 24 hours. The cultivation was continued for additional 24 hours, and the culture supernatant was then collected. The collected culture supernatant was filtrated through 0.45-micron filter (Millipore) to prepare a vGL2 virus supernatant stocks, which were stored at -80°C until use.

### (3) Estimation of the titer of the virus supernatant

The titer of the supernatant was measured by using NIH/3T3 cells (ATCC CRL-1658) according to a standard method [J. Virol., 62:1120-1124 (1988)]. Briefly, 2,000 NIH/3T3 cells per well were cultured overnight in DMEM containing 10% calf serum (CS, Gibco) in a 6-well tissue culture plate (Iwaki Glass). Serial dilutions of the virus supernatant and hexadimethrine bromide (polybrene, Aldrich) to a final concentration of 7.5 µg/ml were then added to each well. After incubation at 37°C for 24 hours, the medium was exchanged for DMEM containing G418 (Gibco) to a final concentration of 0.75 mg/ml and 10% CS, and the incubation was further continued. G418-resistant colonies were stained with crystal violet after 10-12 days, and the number was scored. The number of infections particles contained in 1 ml of the supernatant (cfu/ml), i.e., the titer of the virus supernatant, was calculated by multiplying the number of colonies per well by the dilution rate of the virus supernatant. The amount of the virus supernatant to be used in the experiment hereinbelow was determined based on the titer.

### (4) Transfer of a foreign GFP gene into murine testicular cells

30 mg/kg of busulfan was administered intraperitoneally to MCH (ICR) male mice of four weeks old (four mice per group). The mice were divided into four groups as follows before inoculating a virus. A group into which the virus is inoculated three times, 14, 17 and 20 days after administration (group 1); a group into which the virus is inoculated three times, 21, 24 and 27 days after administration (group 2); a group into which the virus is inoculated three times, 28, 31 and 34 days after administration (group 3); and a group into which the virus is inoculated three times, 35, 38 and 41 days after administration (group 4). Viral inoculation was conducted in a manner in which 50 µl each of the vGL2 virus supernatant (1 x 10⁴ cfu/ml) was inoculated into each of the testes. A pair of testes were removed from one mouse from each group 4, 11, 18 and 25 days after the final viral inoculation. Testicular cell preparations from the testes were subjected to detection of the GFP gene in the cells by a PCR analysis and FACS analysis of the GFP gene product.

### (5) FACS analysis of the expression of the GFP gene in murine testicular cells

The pair of testes were collected from one mouse in Example 2-(4) and tunicae of the testes were removed. The testes were then homogenized using a homogenizer. 1 ml of 0.25% trypsin/EDTA solution was added to the homogenate to dissociate the cell masses. A 30-micron mesh was used to remove remaining cell masses to prepare a testicular cell preparation. The thus-obtained testicular cell preparation was subjected to FACS analysis for examining the expression of the GFP gene. FACSVantage was used for the analysis measuring the fluorescence from the GFP at an excitation wavelength of 488 nm and an emission wavelength of 515-545 nm.

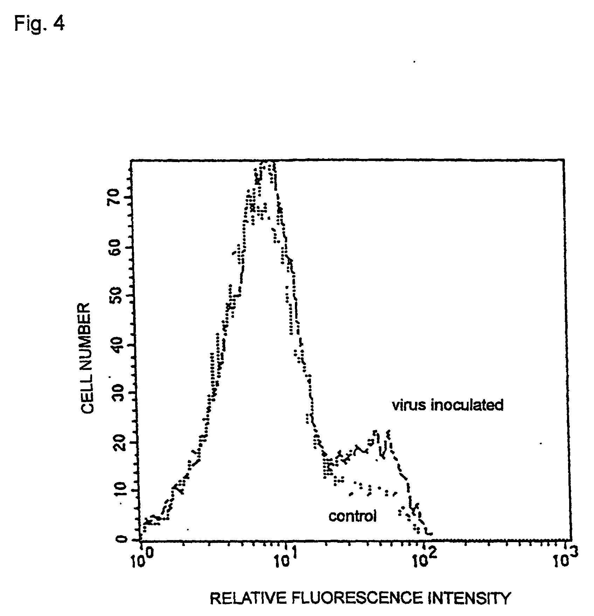

Analytical results were obtained for the testicular cell preparation from the group (3) obtained 25 days after the viral inoculation. Also, results were obtained for a testicular cell preparation from a mouse at the same age without a viral inoculation after the busulfan administration (which was analyzed as a control). These results are shown in Figure 4. For the preparation with the viral inoculation, there exists an area representing the fluorescence from the GFP, which is not observed for the control (the area ranging from 30 to 100 of the relative fluorescence intensity of the GFP indicated on the horizontal axis in the figure). This confirms the expression of the transferred GFP gene in the testicular cells.

### (6) Detection of the GFP gene transferred into murine testicular cells

Chromosomal DNAs were prepared from several testicular cell preparations selected from those prepared in Example 2-(5). Approximately 1 x 10⁶ cells from the testicular cell preparations were suspended in 700 µl of DNA extraction solution (10 mM Tris-HCl (pH 8.0), 100 mM NaCl, 10 mM EDTA, 39 mM DTT and 2% SDS). 35 µl of proteinase K (10 mg/ml, Merck) was added to the suspension. After the mixture was incubated, at 37°C overnight, 2 µl of ribonuclease A (20 mg/ml, Sigma) was added to the mixture and the incubation was continued at 37°C for additional two hours. The reaction was stopped by phenol-chloroform extraction; ethanol precipitation was carried out to recover a DNA, which was then dissolved in 100 µl of TE buffer (10 mM Tris-HCl (pH 8.0), 1 mM EDTA) to obtain a chromosomal DNA solution.

A PCR analysis was used to confirm the chromosomal integration of the GFP gene resulted from the infection of the cells with the vGL2 virus. A PCR reaction mixture which contained the chromosomal DNA solution as well as oligonucleotides GFP-13 and GFP-16R synthesized based on the GFP gene sequence (the base sequences of the oligonucleotides GFP-13 and GFP-16R are shown in SEQ ID NOS: 1 and 2 of the Sequence Listing, respectively) was prepared. The mixture was subjected to a reaction of 94°C for 1 minute and then 30 cycles of 94°C for 30 seconds - 63°C for 30 seconds - 72°C for 30 seconds. TaKaRa Tag (Takara Shuzo) was used for the PCR and the reaction mixture was prepared by using the attached reaction buffer. Also, chromosomal DNA solutions similarly prepared from NIH/3T3 cells infected with the vGL2 virus and a testicular cell preparation from a mouse at the same age without a viral inoculation after the busulfan administration were used as a positive control and a negative control, respectively.

As a result, an amplification of a 234-bp DNA fragment derived from the GFP gene was observed for the following chromosomal DNAs as shown in Table 5. One prepared from the testicular cells from the group 2 obtained 18 days after the final viral inoculation; one prepared from the testicular cells from the group 3 obtained 4 and 25 days after the final viral inoculation; and one prepared from the testicular cells from the group 4 obtained 4 and 18 days after the final viral inoculation

These results demonstrate that a foreign gene, the GFP gene, is integrated into the chromosome with high efficiency in a group inoculated with the virus 4-5 or 5-6 weeks after the busulfan administration. Thus, it revealed that it is important to carry out several viral inoculations around five weeks after the busulfan administration. This period corresponds to the restoring period for the decreased testicular cells as demonstrated in the preliminary experiments described in Example 1.

The results of Examples 2-(4) to (6) indicate that the following is important in order to integrate a gene of interest into the chromosome of the testicular cells in vivo. The gene of interest is incorporated into a vector capable of integrating. It is then inoculated during a period in which testicular cells are restoring from a damage due to a drug.

**Table 5**

| Detection of the GFP gene by PCR analysis | | |
|---|---|---|
| Administration group | Days after the final viral inoculation | Results of PCR assay |
| Group 1 | 18 | - |
| Group 2 | 11 | - |
| | 18 | + |
| Group 3 | 4 | + |
| | 11 | - |
| | 18 | - |
| | 25 | + |
| Group 4 | 4 | + |
| | 11 | - |
| | 18 | + |
| Positive control | | + |
| Negative control | | - |

| | | |
|---|---|---|
| +: A 234-bp fragment was amplified. | | |
| -: A 234-bp fragment was not amplified. | | |

### Example 3

### Transfer of the GFP gene into murine epididymal spermatids and its expression

### (1) Transfer of the GFP gene into murine epididymal spermatids

30 mg/kg of busulfan was administered intraperitoneally to sixteen MCH (ICR) male mice of four weeks old. 50 µl each of the vGL2 virus supernatant (1 x 10⁴ cfu/ml) was inoculated into each of the testes 28, 31 and 34 days after administration. Epididymes were removed from four mice 28, 35, 42 and 49 days after the viral inoculation to prepare sperm fractions. FACS was used to measure the fluorescence from the GFP gene product and to collect cells from fluorescence-positive fractions. Also, a PCR analysis was used to detect the GFP gene in the cells from the fluorescence-positive fractions.

### (2) Analysis of the expression of the GFP gene in murine epididymal spermatids

The murine epididymes as described in Example 3-(1) was minced with scissors into small pieces in a 2% penicillin/streptomycin solution. A 30-micron mesh was then used to remove cell masses to prepare sperm fractions. Expression of the GFP gene in the sperm fractions was analyzed using FACS according to the procedure as described in Example 2-(5). GFP-positive cell fractions were sorted at that time.

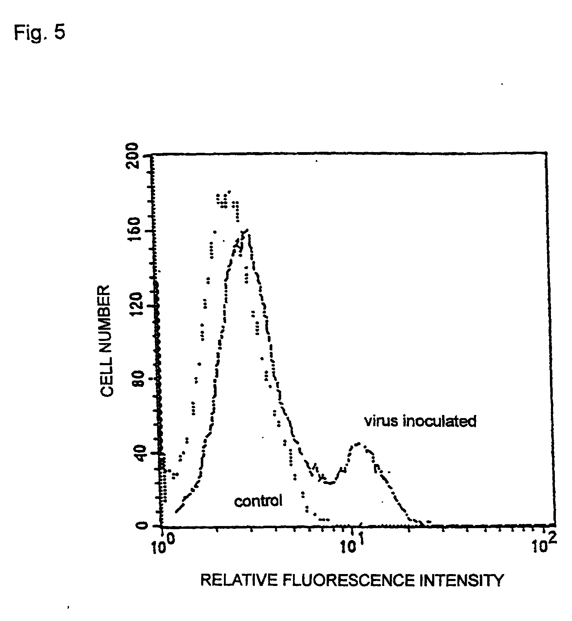

Analytical results were obtained for the sperm fractions isolated from a mouse 42 days after the viral inoculation. Also, results were obtained for a testicular cell preparation from a mouse at the same age without a viral inoculation after the busulfan administration, which was analyzed as a control. These results are shown in Figure 5. As shown in the figure, the existence of sperms expressing the GFP in an epididymis removed from a mouse 42 days after the viral inoculation (the area ranging from 8 to 20 of the relative fluorescence intensity of the GFP indicated on the horizontal axis in the figure) was confirmed.

### (3) Detection of the GFP gene in murine epididymal spermatids

Approximately 1 x 10⁵ cells from the GFP-positive sperm sorted by FACS in Example 3-(2) were suspended in 700 µl of DNA extraction solution (10 mM Tris-HCl (pH 8.0), 100 mM NaCl, 10 mM EDTA, 39 mM DTT and 2% SDS). 35 µl of proteinase K (10 mg/ml) was added to the suspension. After incubation at 37°C overnight, 2 µl of ribonuclease A (20 mg/ml) was added to the mixture and the incubation was continued at 37°C for additional two hours. The reaction was stopped by phenol-chloroform extraction; ethanol precipitation was carried out to recover a DNA, which was then dissolved in 30 µl of TE buffer to obtain a chromosomal DNA solution.

The GFP gene in the chromosomal DNA solution was detected according to the procedure as described in Example 2-(5). As a result, an amplification of a 234-bp DNA fragment, which represents the existence of the GFP gene, was observed for the chromosomal DNAs prepared from the sperms obtained 42 and 49 days after the viral administration as shown in Table 6.

**Table 6**

| Detection of the GFP gene by PCR | | |
|---|---|---|
| Days after the final viral inoculation | Groups without a viral administration | Groups with a viral administration |
| 28 | - | - |
| 35 | - | - |
| 42 | - | + |
| 49 | - | + |

| | | |
|---|---|---|
| +: A 234-bp fragment was amplified. | | |
| -: A 234-bp fragment was not amplified. | | |

## Claims

1. A method for transferring a gene into a vertebrate testicular cell comprising decreasing a testicular cell number and inoculating a recombinant virus carrying a foreign gene into a testis.

2. The method for transferring a gene according to claim 1, wherein decreasing the testicular cell number is conducted by a procedure selected from the group consisting of a chemical procedure, a physical procedure and a biological procedure.

3. The method for transferring a gene according to claim 2, wherein an alkylating agent is used.

4. The method for transferring a gene according to any one of claims 1 to 3, wherein inoculating the recombinant virus into the testis is conducted during a restoring period for the testicular cells.

5. The method for transferring a gene according to any one of claims 1 to 4, wherein a recombinant virus capable of integrating a gene into a chromosome is inoculated.

6. The method for transferring a gene according to claim 5, wherein the recombinant virus is made using a replication-defective virus vector.

7. The method for transferring a gene according to claim 5 or 6, wherein a recombinant retrovirus is inoculated.

8. A vertebrate in which a foreign gene is transferred into a testicular cell by the method according to any one of claims 1 to 7.

9. A testicular cell with a transferred foreign gene obtained from the vertebrate according to claim 8.

10. The testicular cell according to claim 9, selected from the group consisting of a spermatogonium, a spermatocyte, a spermatid and a sperm.

11. A vertebrate with a transferred foreign gene obtained by mating the vertebrate according to claim 8.

12. A vertebrate with a transferred foreign gene obtained from a fertilized ovum artificially produced using the testicular cell according to claim 9 or 10.
